# EUROPEAN PATENT SPECIFICATION

(11) **EP 3 212 058 B1**
(45) Date of publication and mention of the grant of the patent: **17.02.2021**
(21) Application number: 15808482.2
(22) Date of filing: 27.10.2015
(51) Int. Cl.: A61B 3/032, A61B 3/036

(54) **PICTURE FOR DUOCHROME TEST OF A PERSON WITH ASTIGMATISM WITH KNOWN ANGLE OF CYLINDER**
BILD FÜR DUOCHROMEN TEST EINER PERSON MIT ASTIGMATISMUS MIT BEKANNTEM ZYLINDERWINKEL
IMAGE POUR TEST DUOCHROME D'UNE PERSONNE SOUFFRANT D'ASTIGMATISME AVEC UN ANGLE CONNU DE CYLINDRE

(30) Priority: 28.10.2014 SK 500652014
(43) Date of publication of application: 06.09.2017
(73) Proprietor: Prvá Optická, S.r.o., 911 01 Trencín (SK)
(72) Inventor: URBÁNEK, Peter, 911 01 Trencín (SK); WAGNER, Jaroslav, 779 00 Olomouc (CZ); PLUHÁCEK, Frantisek, 796 04 Prostejov (CZ); PALMAN, Ivan, 911 08 Trencín (SK)
(74) Representative: Kubinyi, Peter
(86) International application number: PCT/SK2015/050012
(87) International publication number: WO 2016/068813

(56) References cited:
- EP-A1- 1 470 777
- US-A1- 2006 152 675

## Description

### Technical Field

The invention relates to a device comprising a display configured to display a picture for duochrome test of a person with astigmatism with known angle of cylinder

### Background Art

Duochrome test is based on physical principle of different refraction of light for different wavelengths.

Pictures for douchrome tests of a person with astigmatism and myopia or hyperopia are usually separate pictures for subjective adjustment of refractive error of the human eye. Currently known duochrome pictures are separately for fine spherical adjustment and separately for astigmatic adjustment of cylindrical correction.

Duochrome pictures comprise two color fields, usually red and green or red and blue. Each color field comprises identical geometrical patterns, provided generally in black color. The human eye qualitatively evaluates difference of the visus in each field against different background colors. Black geometrical pattern viewed against red background is projected in different distance from the retina than the same pattern viewed against green background.

Color-inverted duochrome pictures are used as well, what means that the background color is black and geometrical patterns in one part of the picture are red and in the other part are green.

US 2006/0152675 A1 discloses an optometric apparatus and an optometric method which perform an accurate eye examination on people who have a wide range of refractive powers with astigmatism, myopia or hyperopia, and which are also applicable especially to those with mixed astigmatism. The apparatus performs eye examination by prompting a subject to view test symbols displayed on a computer screen by one of the right and left eyes at a time. The system includes also hyperopia and myopia determination means which displays test symbols for determining hyperopia or myopia in two orthogonal orientations selected based on the determined astigmatic axis angle. The test symbols for determining hyperopia and myopia have a rectangular frame divided into equal right and left areas, where in preferred embodiment, the left area has a red background and the right area has a blue background. The test symbols are used as the first, second, third and fourth hyperopia and myopia determination charts. The third chart includes the red area with straight lines oriented corresponding to an astigmatic angle and the blue area with straight lines oriented corresponding to an angle orthogonal to the astigmatic axis angle.

Disadvantages of the state of the art are that, the picture for spherical adjustment and the picture for cylindrical adjustment are used separately.

Aim of the ophtalmologist or optometrist is to make subjective fine adjustment of refractive error of the human eye, both spherically and cylindrically. When separate pictures are used for fine adjustment, it is not possible to make evaluation according to response of the patient, whether to change spherical or cylindrical component of refractive error, because the influence of the change of spherical or cylindrical component of refractive error on qualitative difference of the visus is simultaneous. When separate pictures are used, it is hard to evaluate, whether with fine adjustment of subjective refractive error, spherical or cylindrical value is to be changed.

### Disclosure of Invention

Disadvantages of the background art are eliminated by a device comprising a display configured to display a picture for duochrome test of a person with astigmatism with known angle of cylinder according to Claim 1, by means of which ametropic eye of the person with astigmatism with known angle of cylinder can be simultaneously subjectively adjusted spherically and astigmatically.

The term douchrome test, or duochrome picture within the meaning of this invention is known and clear to the person skilled in the art, though in the strict sense it could be said, that this relates to trichrome picture, because such picture actually comprises three colors, i.e. red, green (alternatively blue), and black.

The eye compares at least pairs of parallel lines placed against the background of two different colors, whereby other pairs of parallel lines are oriented perpendicular to the first parallel lines also against background of the same two colors.

One pairs of parallel lines oriented according to axis of cylinder of the person with astigmatism allow for cylindrical fine subjective adjustment of refraction values of the eye ± 0.25 of cylindrical diopter, even also ± 0.125 of cylindrical diopter.

Other pairs of parallel lines oriented perpendicularly to the axis of cylinder of the person with astigmatism allow for simultaneous spherical fine adjustment of refraction values of the eye ± 0.25 of spherical diopter, even also ± 0.125 of spherical diopter.

Design of the picture according to this invention, provides for the possibility to use it for testing of correct spherical and cylindrical refraction values of directly prepared glasses.

With this single picture according to this invention, it is also possible to test spherical and cylindrical changes of the refraction of the eye on long term basis, in order of months or years.

It is advantageous to create the picture on the display of the device, which is controlled by a software, which by entering of input variables allows to change the angle of orientation of the lines, and/or thickness of the lines, and/or distance of the lines, and/or length of the lines according to examination distance and according to determined angle of cylinder of the person with astigmatism.

If the picture is provided in such way, efficiency of use of the picture extremely increases, as the picture can instantly be adjusted for specific needs of the examination.

It is also advantageous to create the picture by photo process and place it on the viewing plane of other optotypes of the ophtalmologic device for measuring objective and also subjective refraction of the human eye, which, the picture, can be rotated according to the angle of cylinder determined by the very same ophtalmologic device. Known device is commonly used auto kerato-refractometer with built-in optotype charts on its viewing plane.

### Brief Description of Drawings

The invention is closely explained by the drawing. In order to better explain the invention, the figure contains two-dimensional x, y cartesian coordinate system having its centre between two parts of the picture, which, as will be described further, are in actual use colored. This cartesian system is introduced in the picture for the purpose of detailed description of this invention and it is not necessary for actual use of the picture in the examination, thus the picture is in praxis used without it.

### Mode(s) for Carrying Out the Invention

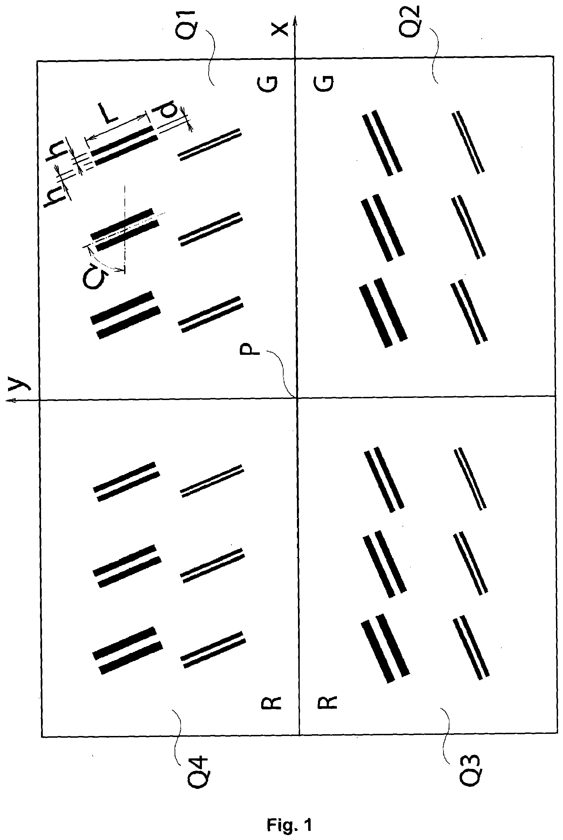

The picture according to Fig. 1 comprises two parts R and G. Actually, one part R can be red and the other part G green (or blue according to the examination standards that can differ), in the case of the inverted picture, i.e. color lines on black background, parts R and G are substantially solid black field, i.e. background. The picture is in Fig. 1 divided, by cartesian coordinate system having the center P, into quadrants Q1, Q2, Q3 and Q4. When such divided, the parts R and G are colors of the background in respective half-planes in relation to y axis. In the case of inverted picture, the parts R and G are meant as half-planes only, without any reference to their color.

Each part R and G comprises geometric pattern, which falls within the scope of definitions introduced in the Disclosure of Invention and the Claims, where as an example according to Fig. 1, 6 different pairs of parallel lines are used, so that
- 6 different pairs of parallel lines in the first quadrant Q1 and the fourth quadrant Q4 are identical and are identically oriented according to known angle Ω of cylinder of the person with astigmatism,
- 6 different pairs of parallel lines in the second quadrant Q2 and the third quadrant Q3 are identical, whereas
- 6 different pairs of parallel lines in the third quadrant Q3 were created by rotation of 6 pairs of parallel lines from the fourth quadrant Q4 by 90 ° around their respective geometrical centres and translation to this third quadrant Q3, and
- 6 different pairs of parallel lines in the second quadrant Q2 were created by rotation of 6 pairs of parallel lines from the first quadrant Q1 by 90° around their respective geometrical centres and translation to this first quadrant Q1.

Number of *n*-tuple, herein pairs, is as an example given as 6. However, in general applies that it concerns at least one *n*-tuple.

Following examples of embodiments of the invention are given for particular values of the examination (viewing) distance referred to as *s,* what is a distance of the cornea of the examined eye from the testing picture. Mentioned particular values of the examination distances are only illustrative, and these are distances commonly used in examination praxis. Present examples of embodiments do not limit scope of the invention to these particular values in any way.

The examination distance s is mostly for long distances and is, for example, defined by standard EN ISO 8596, and is *s* = 4 m, at the minimum. However minimum of *s* = 5 m is applied in praxis.

The examination for short distances or medium distances is also possible, what is defined, for example, by standard DIN 58220-5:2900-11. For short distance (reading) the distances are *s* = 0.4 m, or 0.333 m, or 0.25 m. Medium distances are the distances *s* = 1 m, or 0.667 m, or 0.55 m.

### Example 1

Given is geometric picture according to Fig. 1

There is known angle Ω of cylinder of the person with astigmatism, Ω = 67°. For the *n*-tuple lines composing the geometric picture *n* = 2 is given, i.e. this concerns pairs of parallel lines. Black color is applied in this example of embodiment.

The examination distance *s* = 5 m. The picture in this example of embodiment, has 196 mm width and 147 mm height, what corresponds to display of iPad®2 device, for example.

Dimensions and distance of the lines in *n*-tuple, in this example in pairs, i.e. thickness h of the line, distance d of the lines in the pair at the view angle *α* are following:
- *α* = 0.5 minutes, then h = 0.727 mm, then d = 0.727 mm
- *α* = 0.63 minutes, then h = 0.916 mm, then d = 0.916 mm
- *α* = 0.8 minutes, then h = 1.164 mm, then d = 1.164 mm
- *α* = 1.0 minute, then h = 1.454 mm, then d = 1.454 mm
- *α* = 1.25 minutes, then h = 1.818 mm, then d = 1.818 mm
- *α* = 1.6 minutes, then h = 2.327 mm, then d = 2.327 mm

Width of the picture is hypothetically equidistantly divided to 8 parts by 24.5 mm, from the left edge to geometrical centre of the first pair of lines, from geometrical centre of the first pair to the second, from the second to the third, from the third to y axis, from y axis to the fourth, from the fourth to the fifth, from the fifth to the sixth and from the sixth to the right edge.

Similar applies to the spacing in height, which is hypothetically divided to 6 parts by 24.5 mm, from the upper edge to geometrical centre of the first pair of lines, from the centre of the first pair to the second, from the second to x axis, from x axis to the third, from the third to the fourth and from the fourth to the lower edge.

Geometrical centres of neighboring pairs of lines in horizontal as well as vertical direction are in mutual distances of 24.5 mm.

Length of the lines in this example of embodiment is L = 19 mm.

### Example 2

Given is geometric picture according to Fig. 1

There is known angle Ω of cylinder of the person with astigmatism, Ω = 67°. For the *n*-tuple lines composing the geometric picture *n* = 2 is given, i.e. this concerns pairs of parallel lines. Black color is applied in this example of embodiment.

The examination distance *s* = 0.4 m. The picture in this example of embodiment, has dimensions 106 mm x 59.5 mm, what corresponds to display dimensions of Samsung®S4 device, for example.

Substantially bigger size of display than required just for the picture itself is advantageous.

It is derived from triangle similarity that sufficient dimensions of the picture for distance *s* = 0.4 m, are width 15.68 mm and height 11.76 mm. In general, display size of Samsung®S4 is even advantageous, because the effect of physics principle of different refraction of light for different wavelengths increases herewith.

Dimensions and spacing of the lines in *n*-tuple, in this example in pairs , i.e. thickness h of the line, distance d of the lines in the pair at the view angle *α* are following:
- *α* = 0.5 minutes, then h = 0.058 mm, then d = 0.058 mm
- *α* = 0.63 minutes, then h = 0.073 mm, then d = 0.073 mm
- *α* = 0.8 minutes, then h = 0.093 mm, then d = 0.093 mm
- *α* = 1.0 minute, then h = 0.116 mm, then d = 0.116 mm
- *α* = 1.25 minutes, then h = 0.145 mm, then d = 0.145 mm
- *α* = 1.6 minutes, then h = 0.186 mm, then d = 0.186 mm

Geometrical centres of neighboring pairs of lines in horizontal as well as vertical direction are in mutual distances of 1.96 mm.

Length of the lines in this example of embodiment is L = 1.52 mm.

### Example 3

Given is geometric picture according to Fig. 1

There is known angle Ω of cylinder of the person with astigmatism, Ω = 67°. For the *n*-tuple lines composing the geometric picture *n* = 2 is given, i.e. this concerns pairs of parallel lines.

This example of embodiment discloses inverted picture.

What means:
- in the half-plane left of y axis, in quadrants Q3 and Q4, the color of the background R is chosen black;
- in the half-plane right of y axis, in quadrants Q1 and Q2, the color of the background G is chosen black;
- 6 different pairs of parallel green lines in quadrant Q1 and red lines in quadrant Q4 are identical;
- 6 different pairs of parallel green lines in quadrant Q2 and red lines in quadrant Q3 are identical.

In other words, in this example of embodiment, color part is composed at least of one *n*-tuple line.

The examination distance *s* = 6 m. The picture in this example of embodiment, has width 196 mm and height 147 mm, what corresponds to display dimensions of iPad®2 device, for example.

Dimensions and spacing of the lines in *n*-tuple, in this example in pairs , i.e. thickness h of the line, distance d of the lines in the pair at the view angle *α* are following:
- *α* = 0.5 minutes, then h = 0.872 mm, then d = 0.872 mm
- *α* = 0.63 minutes, then h = 1.1 mm, then d = 1.1 mm
- *α* = 0.8 minutes, then h = 1.396 mm, then d = 1.396 mm
- *α* = 1.0 minute, then h = 1.744 mm, then d = 1.744 mm
- *α* = 1.25 minutes, then h = 2.181 mm, then d = 2.181 mm
- *α* = 1.6 minutes, then h = 2.792 mm, then d = 2.792 mm

Width of the picture is hypothetically equidistantly divided to 8 parts by 24.5 mm, from the left edge to geometrical centre of the first pair of lines, from the centre of the first pair to the second, from the second to the third, from the third to y axis, from y axis to the fourth, from the fourth to the fifth, from the fifth to the sixth and from the sixth to the right edge.

Similar applies to the spacing in height, which is hypothetically divided to 6 parts by 24.5 mm, from the upper edge to geometrical centre of the first pair of lines, from the centre of the first pair to the second, from the second to x axis, from x axis to the third, from the third to the fourth and from the fourth to the lower edge.

Geometrical centres of neighboring pairs of lines in horizontal as well as vertical direction are in mutual distances of 24.5 mm.

Length of the lines in this example of embodiment is L = 22.8 mm.

### Example 4

The picture as is for example disclosed in the above mentioned examples of embodiments, is created on display of a device, which is controlled by a software, for example above mentioned iPad®2 device or Samsung® S4. The picture is then controlled by a software, e.g. software application, which allows to enter input variables, i.e. known angle Ω of cylinder of the person with astigmatism and examination distance *s*. Other parameters of the picture are created and determined by software itself. The user simply enters only known value of the angle Ω of cylinder and value of the examination distance *s*, which the user chooses according to the most convenient conditions for present examination.

The examples of embodiments described above are introduced for explanation of the picture displayed by the device according to this invention. It is clear that value of the angle Ω of cylinder of the person with astigmatism would always be determined for the present examined person, i.e. the value will of course also differ from the value of Ω = 67° given in the examples. Also different examination distance *s* could be chosen, though the examples of embodiments present the most commonly used examination distances.

Regarding dimensions of the picture itself, and dimensions of the lines, such as thickness h, length L, or distance between the lines, these, of course, can also present other values within the ranges as introduced in the Disclosure of Invention and the Claims.

Last but not least, the pairs of lines were chosen for the examples as *n-*tuple, because this is the most advantageous embodiment regarding " readability". However, it is clear that the picture according to the invention can comprise any integer *n*-tuple lines, where n is at least 2, if this would become necessary for the examination needs.

The picture described can, of course, be projected to the examination distance by any suitable projector, i.e. device designed for displaying pictures (transparencies, films, digital images and so) enlarged on screen by front or rear projection. In such way, the picture can be projected for example on a screen or a wall at the examination distance.

It is also possible to use this picture printed on paper or on other known media suitable for printing.

## Claims

1. A device comprising a display configured to display a picture for duochrome test of a person with astigmatism with known angle (Ω) of cylinder, where the picture comprises two color parts (R, G) comprising an n-tuple of parallel lines oriented according to the determined angle (Ω) of cylinder and n-tuple of identical parallel lines perpendicular to the parallel lines oriented according to the determined angle (Ω) of cylinder, **characterized in that,** each of the color parts (R, G) comprises at least one *n*-tuple or is composed of at least one *n*-tuple, where *n* is at least 2, of parallel lines oriented according to the determined angle (Ω) of cylinder of the person with astigmatism and identical parallel lines perpendicular to the parallel lines oriented according to the determined angle (Ω) of cylinder, whereas line thickness (h) in the *n*-tuple is 0.035 to 6 mm, distance (d) between lines in the *n*-tuple is 0.035 to 6 mm, distance between two geometric centres of neighboring *n*-tuple lines is at least equal to the length (L) of the lines and the length (L) of each line in *n*-tuple is at least 0.105 mm.

2. The device according to claim 1, wherein the display configured to display the picture is controlled by a software, which by entering of input variables allows to change the angle of orientation of the lines, and/or thickness (h) of the lines, and/or distance (d) of the lines, and/or length (L) of the lines.

3. The device according to claim 1, wherein the device is an ophthalmologic device for measuring objective and also subjective refraction of the human eye and the picture is configured to be placed on the viewing plane of other optotypes of the ophtalmologic device.

4. The device according to claim 1, wherein the device is a projector for a transparency or film.

## Patentansprüche

1. Einrichtung, die ein Display aufweist, das zur Abbildung eines Bildes für einen duochromatischen Monokulartest eines Astigmatikers mit einem bekannten Winkel (Ω) eines Zylinders eines Astigmatikers konfiguriert ist, wo dieses Bild zwei farbige Teile (R, G) aufweist, die ein n-Tupel von parallel laufenden Linien, die laut dem ermittelten Winkel (Ω) eines Zylinders orientiert sind, und ein n-Tupel von identischen parallel laufenden Linien aufweisen, die zu den parallel laufenden Linien senkrecht sind, die laut dem ermittelten Winkel (Ω) eines Zylinders orientiert sind, **dadurch gekennzeichnet, dass** jeder der farbigen Teile (R, G) mindestens ein n-Tupel aufweist oder durch mindestens ein n-Tupel von parallel laufenden Linien gebildet wird, wobei n mindestens 2 ist, das mit dem ermittelten Winkel (Ω) eines Zylinders eines Astigmatkers übereinstimmend orientiert ist, und identische parallel laufende Linien aufweist, die zu den parallel laufenden Linien senkrecht sind, die laut dem ermittelten Winkel (Ω) eines Zylinders orientiert sind, wobei die Dicke (h) von Linien im n-Tupel 0,035 bis 6 mm ist, Abstand (d) von Linien im n-Tupel 0,035 bis 6 mm ist, Abstand von zwei geometrischen Mittelpunkten von benachbarten n-Tupeln von Linien mindestens der Länge der Linien (L) gleich ist und die Länge (L) von jeder Linie im n-Tupel mindestens 0,105 mm ist.

2. Einrichtung nach dem Anspruch 1, wo das Display, das zur Abbildung eines Bildes konfiguriert ist, durch eine Software gesteuert wird, die ermöglicht, bei einer Eingabe von Eingangsvariablen Winkel der Orientierung von Linien und/oder Dicke (b) von Linien, und/oder Abstand (d) von Linien, und/oder Länge (L) von Linien zu ändern.

3. Einrichtung nach dem Anspruch 1, wo die Einrichtung eine ophthalmologische Einrichtung zur Messung einer objektiven sowie subjektiven Refraktion eines menschlichen Auges ist und das Bild zur Platzierung in einer Abbildungsebene von sonstigen Optotypen von dieser ophthalmologischen Einrichtung konfiguriert ist.

4. Einrichtung nach dem Anspruch 1, wo die Einrichtung ein Projektor für eine transparente Folie oder Film ist.

## Revendications

1. Dispositif équipé d'un écran configuré pour visualiser la figure pour un test duochrome monoculaire d'astigmatisme avec l'angle (Ω) du cylindre connu, où cette figure comprend deux parties colorées (R, G) avec un n-uplet de lignes parallèles orientées par rapport à l'angle (Ω) du cylindre constaté, et un n-uplet de lignes parallèles identiques, perpendiculaires aux lignes parallèles orientées par rapport à l'angle (Ω) du cylindre constaté, **caractérisé en ce que** chacune des parties colorées (R, G) comprend au moins un n-uplet ou est représentée par ou moins n-uplet, où n égale au moins 2, de lignes parallèles orientées vers la même direction que l'angle (Ω) du cylindre détecté, et lignes identiques parallèles perpendiculaires aux lignes parallèles orientées par rapport à l'angle (Ω) du cylindre détecté, tandis que l'épaisseur (h) des lignes dans le n-uplet est entre 0,035 et 6 mm, la distance (d) des lignes dans le n-uplet est entre 0,035 et 6 mm, la distance des deux centres géométriques des n-uplets de lignes voisines est au moins égale à la longueur de lignes (L) et la longueur (L) de chaque ligne dans le n-uplet est au moins 0,105 mm.

2. Dispositif selon la revendication 1, où l'écran configuré pour afficher la figure est contrôlé par un logiciel qui permet, lors de l'introduction des variables d'entrée, de modifier l'angle d'orientation des lignes et/ou l'épaisseur (b) des lignes et/ou la distance (d) des lignes et/ou la longueur (L) des lignes.

3. Dispositif selon la revendication 1, où le dispositif est un appareil ophtalmologique conçu pour mesurer la réfraction objective ainsi que subjective de l'œil humain, où la figure est configurée par rapport à sa position dans le plan d'imagerie des autres optotypes de cet appareil ophtalmologique.

4. Dispositif selon la revendication 1, où le dispositif est un projecteur pour une feuille transparente ou un film.
